# EUROPÄISCHE PATENTSCHRIFT

(11) **EP 1 907 023 B1**
(45) Veröffentlichungstag und Bekanntmachung des Hinweises auf die Patenterteilung: **16.11.2011**
(21) Anmeldenummer: 06764137.3
(22) Anmeldetag: 12.07.2006
(51) Int. Cl.: A61L 27/18, A61L 31/06

(54) **RESORBIERBARE POLYETHERESTER ZUR HERSTELLUNG VON MEDIZINISCHEN IMPLANTATEN**
RESORBABLE POLYETHER ESTERS AND USE THEREOF FOR PRODUCING MEDICAL IMPLANTS
POLYETHERESTERS RESORBABLES ET LEUR UTILISATION POUR PRODUIRE DES IMPLANTS MEDICAUX

(30) Priorität: 15.07.2005 DE 102005033101
(43) Veröffentlichungstag der Anmeldung: 09.04.2008
(60) Teilanmeldung: 10178257.1
(73) Patentinhaber: Evonik Röhm GmbH, 64293 Darmstadt (DE)
(72) Erfinder: BUCHHOLZ, Berthold, 55437 Ockenheim (DE); ENDERLE, Anja, 64367 Mühltal (DE)
(86) Internationale Anmeldenummer: PCT/EP2006/064128
(87) Internationale Veröffentlichungsnummer: WO 2007/009919

(56) Entgegenhaltungen:
- EP-A- 0 295 055
- EP-A1- 0 108 933
- WO-A-2004/007588
- WO-A-2004/009664
- WO-A-2005/011770

## Beschreibung

Die vorliegende Erfindung betrifft resorbierbare Blockcopolymere mit Polyether- und Polyestereinheiten, im Weiteren Poly(etherester) genannt, und deren Verwendung zur Herstellung von chirurgischen oder therapeutischen Implantaten für den menschlichen oder tierischen Organismus. Die erfindungsgemäßen Blockcopolymere und daraus hergestellte Implantate zeichnen sich durch eine verbesserte Resorptionskinetik bei gleichzeitig hoher mechanischer Festigkeit aus. Im Rahmen der Erfindungsbeschreibung wird auf die Herstellung und die Reinigung der erfindungsgemäßen Blockcopolymere eingegangen.

### Stand der Technik

In der WO 2004/009664 wird ein System zur Verabreichung von Wirkstoffen beschrieben, welches ein Hydrogel beinhaltet, das aus Cyclodextrin, einem amphiphilen Copolymer sowie einem Wirkstoff gebildet wird. Das Copolymer besteht dabei aus einem A-Block, der ein Poly(alkylenoxid) umfasst, und aus einem B-Block, der ein Poly(hydroxyalkanoat) umfasst. Die Copolymere der vorliegenden Erfindung sind strukturell unterschiedlich.

In der WO 2004/007588 werden Multiblock-Copolymere beschrieben, die Bereiche eines weichen Präpolymers A und eines harten Präpolymers B umfassen. Die Präpolymere A und B sind miteinander durch multifunktionale Kettenverlängerer verbunden und weisen Strukturen der Formeln (1)-(3) auf Seite 9 auf.

Diese Multiblock-Copolymere unterscheiden sich strukturell wesentlich von den Blockcopolymeren der vorliegenden Erfindung.

In der WO 2005/011770 werden Überzüge für Devices zur Wirkstoffverabreichung beschrieben. Der Überzug beinhaltet ein ABA- oder AB-Blockcopolymer, wobei eine der Einheiten A oder B eine biologische Antwort produziert und die andere Einheit A oder B das Blockcopolymer mit einer strukturellen Funktionalität versieht.

Sowohl die strukturelle Einheit als auch die biologische Einheit können aus einer Liste verschiedener Polymere ausgewählt werden. Spezifisch werden in der WO 2004/009664 nur die Blockcopolymere PEG-PCL-PEG (Formel I) und PCL PEG-PCL (Formel II) genannt:

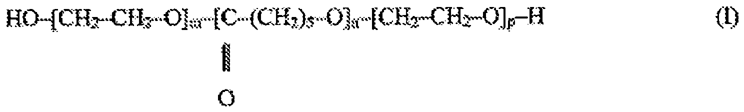

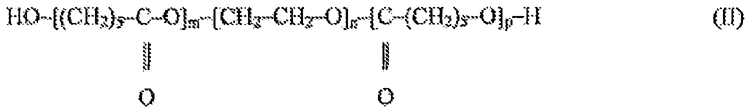

Die vorliegende Erfindung verwendet als vergleichbare "biokompatible Einheit" B nur Polyethylenglykol (PEG), welches mit 0.1 bis 4 Gew.% im Blockcopolymer enthalten ist. Für den A-Block wird reines Polycaprolacton (PCL) nicht verwendet. Epsilon-Caprolacton wird für den A-Block nur im Gemisch mit L Lactid verwendet.

In der EP 0 295 055 werden Multiblock-Polymere beschrieben, die eine der drei folgenden Wiederholungseinheiten aufweisen:

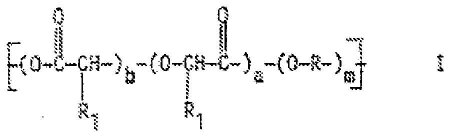

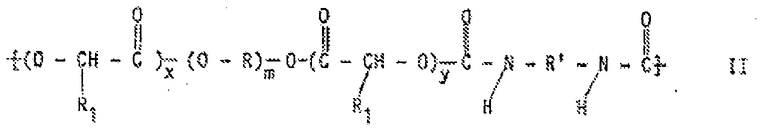

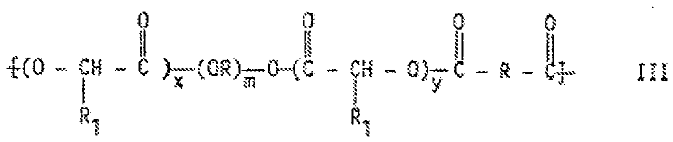

Keine dieser Wiederholungseinheiten findet sich in Blockcopolymeren der Formeln I und II der vorliegenden Erfindung.

In der EP 0 108 933 werden Poly(glykolsäure)poly(oxyethylen)-ABA-Triblockcopolymere beschrieben mit einem Gewichtsanteil des B-Blocks (= Poly(oxyalkylen))von 5-25%. Diese Copolymere werden zur Herstellung von Fasern mit einem ausreichend niedrigen Elastizitätsmodul verwendet.

Die Ansprüche der vorliegenden Erfindung umfassen Blockcopolymere vom AB- oder ABA-Typ, die einen äußerst geringen Anteil an B-Block (Polyether) aufweisen, nämlich 0.1 bis 4 Gew.%.

Resorbierbare Polymere gewinnen zunehmend als Additive in pharmazeutischen Formulierungen oder in bioabbaubaren Implantaten an Bedeutung. Für die verschiedenen technischen Bereiche werden Polymere mit deutlich unterschiedlichen physikalischen und chemischen Eigenschaften benötigt.

So werden als Additive für pharmazeutische Formulierungen u.a. Blockcopolymere mit Polyester- und Polyethereinheiten als Trägermaterialien für Wirkstoffe oder als Materialien für die Mikroverkapselung von Wirkstoffen verwendet. In diesem Fall werden die Polymere bevorzugt über den parenteralen Applikationsweg zugeführt. Im Fall von Trägermaterialien setzt diese Art der Verwendung voraus, dass die Materialien entweder als Pulver, in Lösung oder in Suspension mit den anderen Formulierungskomponenten ohne Qualitätsverluste vermischt werden können. Für Mikroverkapselungen gilt ebenfalls, dass das verwendete Polymer sich gegenüber den weiteren Formulierungsbestandteilen chemisch und physikalisch neutral verhält. Eine weitere Anforderung besteht darin, dass die Mikrokapsel am Zielort den Wirkstoff freisetzt. Naturgemäß scheiden spröde oder feste Materialien in diesen Anwendungen aus. In diesem Bereich haben sich u.a. Poly(etherester) vom AB-Typ, ABA-Typ, BAB-Typ, ABABABAB-Typ als geeignet erwiesen. Als Beispiele seien Polymere mit L- oder D,L-Lactid für den A-Block und Polyethylenglycol für den B-Block genannt.

Neben der Verwendung für den pharmazeutischen Bereich rücken resorbierbare Polymere zunehmend auch in der Chirurgie und chirurgischen Therapie in das Interesse der Fachleute.

Bei der Auswahl der in diesem Bereich als geeignet erscheinenden Materialien müssen neben toxikologischen Eigenschaften im Gegensatz zu den pharmazeutischen Verwendungen gerade die mechanischen Eigenschaften der Materialien berücksichtigt werden. Hier kommen feste Materialien zum Einsatz, die einerseits dem toxikologischen und mechanischen Anforderungsprofil Genüge tun, aber auch Eigenschaften, die für die Herstellung und Verarbeitung notwendig sind. So sollen die Materialien zugänglich sein für thermoplastische Verarbeitungsverfahren, wie Spritzguss, Schmelzpressen, Extrusion oder den Anforderungen von mechanischen Methoden wie spanabhebende Verfahren standhalten. Die diesbezüglich wesentlichen Eigenschaften sind hauptsächliche Festigkeit bei Zug- oder Biegebeanspruchung, sowie die Degradationsgeschwindigkeit.

Die Eigenschaften eines Implantates werden vornehmlich durch den verwendeten Werkstoff bestimmt und weniger durch seine Verarbeitung.

Die resorbierbaren Implantate bieten gegenüber nichtresorbierbaren Materialien wie Metallen den Vorteil, dass sie nach Funktionserfüllung vom menschlichen oder tierischen Körper hydrolytisch abgebaut werden und die Abbauprodukte vom Körper resorbiert werden. Eine Entfernung des Implantats im Rahmen einer Zweitoperation ist somit nicht erforderlich. Ein weiterer Vorteil der resorbierbaren Implantate besteht z. B. in der besseren Verträglichkeit der Materialien, wie sich am Beispiel der Osteosynthese zeigt, wo bei nicht-resorbierbaren Implantaten die Gefahr einer Inaktivitätsatrophie des Knochens besteht, was zu einer erhöhten Gefahr einer erneuten Fraktur des Knochens nach Implantatentfernung führen kann. Die für chirurgische Zwecke interessanten Polymere sollten neben der Resorptionsfähigkeit auch andere Eigenschaften wie hohe Festigkeit aufweisen.

Bekannte resorbierbare Polymere, die sich für chirurgische Implantate u.ä. eignen, sind beispielsweise aliphatische Poly(ester) auf der Basis von Lactid (=3,6-Dimethyl-1,4-dioxan-2,5-dion), Glycolid (= 1,4-Dioxan-2,5-dion), Dioxanon (1,4-Dioxan-2-on), Copolymere aus Lactid/Glycolid mit Trimethylencarbonat (= 1,3-Dioxan-2-on) und epsilon-Caprolacton. Hierbei werden bevorzugt Vertreter mit hohen Molekulargewichten verwendet.
Als Beispiele werden genannt: Poly(L-lactid), Poly(D,L-lactid), Poly(L-lactid-co-D,L-lactid), Poly(D,L-lactid-co-glycolid), Poly(glycolid), Poly(L-lactid-co-glycolid), Poly(glycolid-co-trimethylencaronat), Poly(L-lactid-co-trimethylencarbonat), Poly(D,L-lactid-co-trimethylencarbonat), Poly(L-lactid-co-caprolacton).

Mit den vorstehend genannten Werkstoffen lassen sich resorbierbare Implantate herstellen, welche in ihren mechanischen Eigenschaften ein weites Spektrum abdecken. Beispielsweise weist Poly(L-lactid) bei einer hohen Festigkeit auch eine große Steifigkeit und Sprödigkeit auf, während sich durch Copolymerisation von D,L-Lactid und Trimethylencarbonat Werkstoffe mit zähelastischen Eigenschaften herstellen lassen. Aufgrund einer Abbaugeschwindigkeit von einigen Monaten bis mehreren Jahren eignen sich diese Werkstoffe besonders für Implantate, die entsprechend lang im Körper verbleiben sollen.

Andererseits besteht in der Chirurgie häufig ein bisher nicht befriedigter Bedarf für schneller degradierbare Werkstoffe und Implantate. Insbesondere für pädiatrische Applikationen und für Implantate zur Fixierung von schnell proliferierendem Gewebe werden Werkstoffe benötigt, welche vergleichsweise schnell abgebaut werden, aber dennoch die notwendigen mechanischen Eigenschaften wie Festigkeit, Elastizität, Zähigkeit usw. aufweisen.

Die vorliegende Erfindung leistet einen Beitrag zur Bereitstellung solcher Werkstoffe. Es wurde nämlich überraschend gefunden, dass durch ringöffnende Polymerisation von cyclischen Estern in Gegenwart von mono- oder difunktionalem Poly(ethylenglycol) bei gemäßigten Synthesebedingungen hochmolekulare Blockcopolymere vom AB- oder ABA-Typ in einem einfachen, industriell anwendbaren Maßstab herzustellen sind, die geeignet sind, daraus Implantate mit verbesserten Eigenschaften herzustellen. Diese Polymere sind zudem auch im industriellen Maßstab in einfacher Form z.B. durch Extraktionsverfahren zu reinigen, so dass ihr Reinheitsgrad den Anforderungen für eine Implantation in den Körper genügt. Daneben weisen sie Eigenschaften auf, die es erlauben, die Materialien über einfache thermoplastische Formgebungsmethoden zu Implantaten zu verarbeiten.

### Aufgabe der Erfindung

Aufgabe der vorliegenden Erfindung ist es, Werkstoffe zur Herstellung von medizinischen und chirurgischen Implantaten bereit zu stellen, die gegenüber den aus dem Stand der Technik bekannten Werkstoffen schneller vom menschlichen oder tierischen Organismus abgebaut werden, gleichzeitig jedoch eine hohe mechanische Festigkeit, wie beispielsweise Zugfestigkeit aufweisen.

Eine weitere Aufgabe besteht darin, Werkstoffe zur Herstellung von medizinischen und chirurgischen Implantaten bereit zu stellen, die die für Implantate geeigneten physikalischen Eigenschaften besitzen. Dazu können beispielsweise initiale Festigkeit, Elastizität oder Zähigkeit zählen.

Eine weitere Aufgabe der Erfindung besteht darin, die erfindungsgemäßen Werkstoffe in einem Reinheitsgrad zur Verfügung zu stellen, welcher die Anwendung im menschlichen oder tierischen Körper gestattet. Dabei wird insbesondere auf einen geringen Gehalt an Syntheseausgangsstoffen in dem fertigen Werkstoff Wert gelegt.

Eine weitere Aufgabe der Erfindung besteht darin, Materialien für chirurgische Implantate bereit zu stellen, welche schnell genug resorbiert werden, um in pädiatrischen Applikationen oder als Implantate zur Fixierung von schnell proliferierendem Gewebe verwendet werden zu können.

Eine weitere Aufgabe der Erfindung ist es, ein industriell anwendbares Verfahren zur Herstellung von Rohstoffen für die o.g. Implantate zur Verfügung zu stellen.

Eine weitere Aufgabe der Erfindung ist es, medizinische Implantate und Verfahren zu ihrer Herstellung zur Verfügung zu stellen.

### Beschreibung der Erfindung

Gegenstand der Erfindung sind Blockcopolymere aus Polyestereinheiten und Polyethereinheiten zur Herstellung von resorbierbaren, chirurgischen Implantaten.

Ein bevorzugter Gegenstand der vorliegenden Erfindung sind Implantate, enthaltend ein erfindungsgemäßes Blockcopolymer.

Unter dem Begriff Implantat wird ein (resorbierbarer) Formkörper verstanden, der chirurgisch wie auch mechanisch zur Einbringung in den menschlichen oder tierischen Organismus geeignet ist und toxikologisch unbedenklich ist. Derartige Formkörper können sein: Schrauben, Dübel, Platten, Muttern für Schrauben, Anker, Vliese, Folien, Membrane, Netze usw. Sie können eingesetzt werden zur Fixation von Hartgewebefrakturen, als Nahtmaterialanker, als Wirbelsäulenimplantat, zum Verschließen und Verbinden von Blut- und anderen Gefäßen, als Stent, zum Verfüllen von Kavitäten oder Löchern bei Gewebedefekten usw.

Die erfindungsgemäßen Blockcopolymere eignen sich beispielsweise auch für die Herstellung von drug eluting stents. Dies sind Gefäßstützen zur Einbringung in arterielle Gefäße, die proliferationshemmende Wirkstoffe über einen längeren Zeitraum in das umliegende Gewebe freisetzen zur Verhinderung einer Restenose. Bewährt haben sich hier beispielsweise Wirkstoffe aus der Gruppe der Zytostatika, wie zum Beispiel Paclitaxel.

Die erfindungsgemäßen Implantate lassen sich aus den Werkstoffen durch thermoplastische Formgebungsverfahren zu den für die medizinische Anwendung gewünschten Formen, wie beispielsweise Schrauben, verarbeiten. Geeignet hierfür sind aus dem Stand der Technik per se bekannte Formgebungsverfahren für Thermoplaste, wie Schmelzpressung und vorzugsweise Extrusions- und Spritzgussverfahren. Besonders bevorzugt ist eine Formgebung durch Spritzgussverfahren.

Die für die Spritzgussverarbeitung geeigneten Temperaturen hängen von der genauen Copolymerzusammensetzung ab und liegen im Bereich zwischen 110 und 210 °C. Für Blockcopolymere mit hohem Molekulargewicht und damit hoher Schmelzviskosität werden höhere Verarbeitungstemperaturen benötigt als für Polymere mit vergleichsweise geringem Molekulargewicht. Aufgrund ihrer Kristallinität werden für Blockcopolymere mit einem hohen Anteil an L-Lactid-Einheiten ebenfalls hohe Verarbeitungstemperaturen benötigt.

Wegen der Empfindlichkeit gegenüber hydrolytischer Zersetzung ist es weiterhin vorteilhaft, die Blockcopolymere vor ihrer Verarbeitung zu trocknen sowie die Verarbeitungstemperaturen so niedrig wie möglich zu halten.

Die erfindungsgemäß verwendeten Blockcopolymere sind Polyetherester vom AB-Typ bzw. ABA-Typ.

Dabei steht A für einen Polymerblock mit wiederkehrenden Estereinheiten und B für einen Polymerblock mit wiederkehrenden Ethereinheiten.

Der Polyesterblock A besteht aus n-Bausteinen, die formal auf eine oder mehrere Hydroxycarbonsäuren oder ein Carbonat zurückgehen, bevorzugt auf eine alpha-Hydroxycarbonsäure. Gegebenenfalls kann sich ein Block A formal auch aus mehreren verschiedenen dieser Bausteine aufbauen.

Der Buchstabe B steht für eine Polyethereinheit, deren Repetiereinheiten sich formal aus Ethylenglycol ableiten. Die Repetiereinheiten können m-fach vorhanden sein.

Die erfindungsgemäßen Polymere werden hergestellt, indem an einem Polyethylenglycolblock B mit ein oder zwei freien endständigen OH-Gruppen der oder die Polyesterblöcke A aufgebaut werden. Für Polymere des ABA Typs werden dementsprechend Polyethylenglycolblöcke mit zwei freien endständigen OH-Gruppen verwendet, für Polymere des AB-Typs Polyethylenglycolblöcke mit nur einer freien endständigen OH-Gruppe.

Die erfindungsgemäßen Polyetherester des Typs AB lassen sich durch die allgemeine Formel I darstellen:

Formel I: E-(O-D-CO-)ₙ-(O-CH₂-CH₂-)ₘ-O-F

Die Struktureinheit E-(-O-D-CO-)ₙ- bildet den Block A.

Der Block A ist über eine kovalente Bindung mit dem Block B verknüpft.

D kann für jede der n-Einheiten unabhängig voneinander sein:

-(CH(CH₃)-)ₓ

oder

-(CH₂-)ₓ

oder

-(CH₂-O-CH₂-CH₂-)

oder

-(CH₂-CH₂-CH₂-O-),

x ist 1, 2, 3, 4 oder 5 und
E ist H, Methyl oder Ethyl;
n ist eine integere Zahl größer 1.

Die Struktureinheit -(O-CH₂-CH₂-)ₘ-O-F bildet den Block B.

F ist H, Methyl oder Ethyl;
m ist eine integere Zahl größer 1.

Es sei ausdrücklich darauf hingewiesen, dass AB-Blockcopolymere strukturgleich mit BA-Blockcopolymeren sind, weshalb im Rahmen dieser Erfindungsbeschreibung diesbezüglich keine Differenzierung vorgenommen wird.

ABA-Blockcopolymere sind auf Basis der obigen Definitionen Polymere der allgemeinen Formel II.

Formel II: E(-O-D-CO)ₙ-(O-CH₂-CH₂-)ₘ-O-(CO-D-O-)ₙ-E

mit allen Variablen in der oben genannten Bedeutung und n' ist eine integere Zahl größer 1.

Wie bei Polymeren üblich handelt es sich bei den Angaben n, n' und m um Zahlen, die die statistisch gemittelte Kettenlänge der beiden Blöcke beschreiben. Die genauen Zahlen in jedem Einzelmolekül unterliegen einer statistischen Verteilung.

Die Länge des B-Blocks in den Copolymeren kann im Mittel zwischen 500 und 10000 Dalton liegen. Bevorzugt ist eine gemittelte Blocklänge zwischen 600 und 8000 Dalton, besonders bevorzugt eine gemittelte Blocklänge zwischen 1000 und 8000 Dalton.
Ein kurzes Poly(ethylenglycol)-Fragment, welches durch den hydrolytischen Abbau des Blockcopolymeren freigesetzt wird, kann vom Körper leicht renal ausgeschieden werden. An dieser Stelle sei darauf hingewiesen, dass alle in dieser Beschreibung genannten Intervallsangaben jeweils die Randwerte mit einschließen.

Das gewichtsmäßige Verhältnis vom A- zum B-Block spielt eine wesentliche Rolle für die Eigenschaften der Copolymere und der hieraus hergestellten erfindungsgemäßen Implantate. Je größer der Anteil an B-Block, desto hydrophiler ist der Werkstoff, was positive Effekte im Hinblick auf eine schnelle Resorbierbarkeit (Hydrolyse- oder Abbaugeschwindigkeit) begründet.

Erfindungsgemäß am stärksten bevorzugt sind Polymere mit einem Anteil an B von 0. 1 bis 4 Gew.%. Dies ist von besonderem Vorteil, da Formkörper, welche ein Blockcopolymer mit hoher Dominanz im A-Block enthalten, eine höhere Festigkeit aufweisen. Daher sind Ausführungsformen mit einem Anteil von B von 1 bis 3 Gew.% und insbesondere 0.5 bis 1.5 Gew.% diesbezüglich noch weiter bevorzugt.
Es wurde nämlich überraschenderweise gefunden, dass bei diesen Ausführungsformen mit nur geringen Anteilen des hydrophilen B-Blocks der hydrolytische Abbau gegenüber den entsprechenden reinen Poly(estern) A deutlich beschleunigt wird.

Durch die genannten Angaben zur Länge des Blocks B und dessen Gewichtsanteil am Gesamtpolymeren ergeben sich zwangsläufig die Blocklängen des oder der A-Blöcke und damit auch das Gesamtmolekulargewicht. Bei Triblockcopolymeren des Typs ABA ist davon auszugehen, dass die Länge und der Gewichtsanteil der beiden Blöcke A im Mittel gleich groß sind. Ebenso wird das Gesamtmolekulargwicht des Copolymeren durch das bei der Synthese eingesetzte Molekulargewicht des Ethylenglycols und das Einsatzverhältnis beider Komponenten festgelegt.

An dieser Stelle wird darauf hingewiesen, dass naturgemäß auch über die Länge und den Anteil des Blocks B das Molekulargewicht des finalen Blockcopolymers maßgeblich mitbestimmt werden kann.

Als weiterer wichtiger Parameter für die Charakterisierung der für die erfindungsgemäße Verwendung geeigneten Polymere kann die inhärente Viskosität (i.V.) herangezogen werden. Die inhärente Viskosität der Blockcopolymere kann in einem weiten Bereich variiert werden. Bevorzugt wird eine inhärente Viskosität (gemessen in einem Ubbelhode-Viskosimeter in Chloroform bei 25°C in 0.1 prozentiger Lösung) zwischen 0.1 und 6 dl/g, bevorzugt zwischen 0.5 und 5 dl/g. Besonders bevorzugt sind Werte zwischen 0.6 und 3 dl/g und insbesondere bevorzugt sind Werte zwischen 0.7 und 2.75 dl/g.

Erfindungsgemäß bevorzugt sind Blockcopolymere vom AB- oder ABA-Typ mit folgenden Merkmalen:
- Die inhärente Viskosität (gemessen in Chloroform bei 25°C in 0.1 prozentiger Lösung) liegt zwischen 0.1 und 5.5 dl/g, bevorzugt zwischen 0.5 und 5.0 dl/g.
- Die mittlere Blocklänge im B-Block liegt zwischen 1000 und 8000 Dalton.
- Der Gewichtsanteil des B-Blocks liegt zwischen 0.1 und 4 Gew.%.
- Der Block A weist bevorzugt folgende Esterbausteine auf:
   - Gemische aus statistisch verteiltem (randomisierten) L-Lactid und D,L-Lactid, bevorzugt mit einem molaren Anteil L-Lactid zwischen 60 und 90%,
   - Gemische aus statistisch verteiltem (randomisierten) L-Lactid und Glycolid, bevorzugt mit einem molaren Anteil L-Lactid zwischen 85 und 99%, weiter bevorzugt zwischen 87 und 95%.
   - Gemische aus statistisch verteiltem (randomisierten) D,L-Lactid und Glycolid, bevorzugt mit einem molaren Anteil D,L-Lactid zwischen 50 und 80%.
- Gemische aus statistisch verteiltem (randomisierten) L-Lactid oder D,L-Lactid und epsilon-Caprolacton-Einheiten.
- Gemische aus statistisch verteiltem (randomisierten) L-Lactid oder D,L-Lactid und Dioxanon-Einheiten.
- Gemische aus statistisch verteiltem (randomisierten) L-Lactid oder D,L-Lactid und Trimethylencarbonat-Einheiten.

Stärker bevorzugt sind Blockcopolymere, worin der Block A bevorzugt folgende Esterbausteine aufweist:
- Gemische aus statistisch verteiltem (randomisierten) L-Lactid und D,L-Lactid, bevorzugt mit einem molaren Anteil L-Lactid zwischen 60 und 90%,
- Gemische aus statistisch verteiltem (randomisierten) D,L-Lactid und Glycolid, bevorzugt mit einem molaren Anteil D,L-Lactid zwischen 50 und 80%.

Die erfindungsgemäßen Implantate enthalten bevorzugt einen oder mehrere verschiedene der erfindungsgemäßen Blockcopolymere und daneben keine weiteren Additive, es sei denn Verunreinigungen aus dem Polymerisationsprozess. In einer besonderen Ausgestaltungsform können die Implantate auch Blends von hochmolekularen Blockcopolymeren mit anderen resorbierbaren Materialien, wie beispielsweise resorbierbare Poly(ester) aus der Gruppe Poly(L-Lactid), Poly(D-Lactid), Poly(D,L-lactid), Poly(meso-lactid), Poly(glycolid), Poly(trimethylencarbonat), Poly(dioxanon), Poly(epsilon-Caprolacton), sowie Copolymere aus den entsprechenden Heterozyklen oder Polyethylenglycol enthalten. Bevorzugt werden solche Blends, bei denen die chemische Struktur des A-Blocks im Blockcopolymeren derjenigen im Poly(ester) entspricht. Hierdurch wird eine gute Phasenankopplung im Blend gewährleistet, was für gute mechanische Eigenschaften vorteilhaft ist. Auch können Blends aus verschiedenen Blockcopolymeren zur Anwendung kommen.

Die erfindungsgemäßen Implantate können ein Gewicht zwischen 1 und 10000 mg aufweisen, bevorzugt ein Gewicht zwischen 5 und 5000 mg und besonders bevorzugt ein Gewicht zwischen 10 und 1000 mg.

Die bevorzugte Zugfestigkeit der Implantate gemessen als Formkörper gemäß der Norm ASTM D 638, die erfindungsgemäß aus den oben beschriebenen Polymeren hergestellt werden, beträgt mindestens 70 MPa, bevorzugt 75 bis 95 MPa, besonders bevorzugt 80 bis 88 MPa.

Die bevorzugte Hydrolysegeschwindgkeit (Abbaugeschwindigkeit) der Implantate, bestimmt über die Veränderungen der inhärenten Viskosität an Formkörpern gemäß der Norm ASTM D 638, die erfindungsgemäß aus den oben beschriebenen Polymeren hergestellt werden, beträgt in einem Bad aus einem wässrigen Phosphatpuffer mit pH 7.4 bei einer Temperatur von 37°C nach 6 Wochen mindestens 30%, bevorzugt 40%, besonders bevorzugt 45% bezogen auf den Ausgangswert.

Ein weiterer Gegenstand der Erfindung ist ein Implantat, dadurch gekennzeichnet, dass es ein Blend enthält bestehend aus:
(a) einem erfindungsgemäßen Blockcopolymer und
(b) einem resorbierbaren Polyester A mit wiederkehrenden Einheiten des Lactids, ausgewählt aus L-Lactid, D-Lactid, DL-Lactid oder meso-Lactid, Glycolid, Trimethylencarbonat, epsilon-Caprolacton oder Dioxanon, wobei nur gleichartige oder verschiedene der genannten Einheiten enthalten sein können.

### Herstellung der Blockcopolymere

Copolymere vom AB-Typ können durch ringöffnende Polymerisation von cyclischen Estern in Gegenwart von Poly(ethylenglycol) mit einer freien Hydroxylgruppe und einer nichtreaktiven Endgruppe, bevorzugt sind Methoxy-Endgruppen, synthetisiert werden. Copolymere vom ABA-Typ können in Gegenwart von Poly(ethylenglycol) mit zwei freien Hydroxylgruppen hergestellt werden. In diesem Fall werden die beiden Blöcke A im gleichen Syntheseschritt parallel aufgebaut.

Als Bausteine für die ringöffnende Polymerisation zur Herstellung des Blocks A bzw. der Blöcke A dienen cyclische Ester der allgemeinen Formel III. 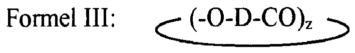 wobei D in jeder der Einheiten -O-D-CO unabhängig voneinander eine der oben genannten Definitionen haben kann.

z ist eine ganze Zahl und mindestens 1, bevorzugt 1 oder 2. Besonders bevorzugt werden dimere cyclische Ester von alpha-Hydroxycarbonsäuren, monomere Lactone oder cyclische Carbonate verwendet.

Bei der Herstellung des Blocks oder der Blöcke A ist es nicht zwingend, dass nur eine Art von Bausteinen nach Formel III verwendet wird. Es können auch Gemische verwendet werden, die sich durch die chemische Natur des Strukturelements D unterscheiden.

Bevorzugte Strukturen nach Formel III, bzw. bevorzugte Moleküle, aus denen die Blöcke A durch ringöffnende Polymerisation gebildet werden, sind a) Gemische aus D- und L-Lactid, wie z.B. racemisches D,L-Lactid, b) Gemische aus L-Lactid und D,L-Lactid, c) Gemische aus L-Lactid und Glycolid, d) Gemische aus D,L-Lactid und Glycolid, e) Gemische aus L-Lactid und epsilon-Caprolacton, f) Gemische aus L-Lactid und Dioxanon, g) Gemische aus L-Lactid und Trimethylencarbonat.

Besonders bevorzugt sind a) L-Lactid und Glycolid, b) L-Lactid und D,L-Lactid, c) D,L-Lactid und Glycolid in den oben genannten Mengenverhältnissen (s. Beschreibung der Polymeren).

Für die Herstellung der Poly(etherester) ist es empfehlenswert, die Rohstoffe in einem hohen Reinheitsgrad einzusetzen. Insbesondere polar-protische Verunreinigungen, wie z.B. Wasser führen zu einem Kettenabbruch während der Polymerisation. Aus diesem Grund ist es empfehlenswert, das Poly(ethylenglycol) vor Einsatz in der Polymerisation zu trocknen.

Für die Synthese wird Poly(ethylenglycol) mit einem oder mehreren der Monomeren bzw. Dimeren nach Formel III gemischt und aufgeschmolzen. Nachdem die Edukte homogen gemischt sind, wird dann der für die Ringöffnungspolymerisation vorgesehene Katalysator zugegeben. Das Reaktionsgemisch wird bevorzugt bei erhöhter Temperatur polymerisiert.

Für die Synthese eignen sich eine Reihe verschiedener Metallkatalysatoren, wie beispielsweise Zinn- oder Zinkverbindungen. Bevorzugt werden Zinn(II)chlorid oder Zinn(II)ethylhexanoat verwendet. Im Hinblick auf die vorgesehene Verwendung im menschlichen oder tierischen Organismus ist es vorteilhaft, eine möglichst geringe Menge Katalysator einzusetzen. Bevorzugt wird eine Konzentration zwischen 30 und 200 ppm, besonders bevorzugt eine Konzentration zwischen 50 und 100 ppm. Dabei bezieht sich die Konzentrationsangabe des Katalysators jeweils auf Gewichtsteile des katalysierenden Metallions bezogen auf die gesamte Reaktionsmasse.

Die Reaktionstemperatur liegt oberhalb der Schmelztemperatur der jeweils eingesetzten Edukte und richtet sich daher nach der Struktur des oder der Monomeren bzw. Dimeren nach Formel III und nach dem Molekulargewicht des eingesetzten Poly(ethylenglycols). Üblicherweise wird in einem Temperaturbereich zwischen 100 und 160°C gearbeitet. Bevorzugt ist ein Bereich zwischen 100 und 140°C, besonders bevorzugt zwischen 110 und 130°C. Bei Polymeren, bei denen eine gute Löslichkeit in organischen Lösungsmitteln angestrebt wird, kann die Reaktionstemperatur auf 150°C bis 170°C eingestellt werden. Durch eine höhere Reaktionstemperatur wird eine gute statistische Verteilung der Comonomer-Einheiten im A-Block bzw. in den A-Blöcken begünstigt. So können z.B. glycolidhaltige Polymere, die sich gut in Aceton lösen, hergestellt werden.

Die erforderlichen Reaktionszeiten richten sich nach der Reaktionsgeschwindigkeit des oder der eingesetzten Monomeren bzw. Dimeren nach Formel III, der Reaktionstemperatur und auch der nach Katalysatorkonzentration und liegen zwischen wenigen Stunden und mehreren Tagen. Bevorzugt ist eine Reaktionszeit zwischen 24 Stunden und 5 Tagen, besonders bevorzugt eine Zeit zwischen 2 und 5 Tagen. Längere Reaktionszeiten bewirken in der Regel einen höheren Umsatz, was wiederum zu einer Reduktion der Konzentration der Edukte im Endprodukt beiträgt.

Zusammenfassend werden die relevanten Reaktionsparameter, nämlich Katalysatormenge, Reaktionstemperatur und Reaktionszeit in Abhängigkeit der eingesetzten Edukte unter den Aspekten möglichst geringer Katalysatorgehalt, gemäßigte Reaktionstemperatur zur Vermeidung von Zersetzungsreaktionen und Verfärbungen im Produkt und möglichst weitgehender Umsetzungsgrad der Monomeren bzw. Dimeren gewählt.

Im Regelfall wird das nach oben angegebener Beschreibung hergestellte Polymer noch einer Reinigungsoperation unterworfen. Da die Ringöffnungspolymerisation der Edukte nach Formel III eine Gleichgewichtsreaktion darstellt, sind im Rohpolymeren noch Spuren an nicht umgesetzten Edukten enthalten, die bei der weiteren Verarbeitung und für die Implantation nachteilig sein können.

Die Reinigung der Polymere kann durch Umfällung aus verschiedenen Lösungsmitteln oder durch Extraktion stattfinden.

Für die Umfällung wird das Rohpolymer in einem mit Fällungsmittel mischbaren Lösungsmittel, z.B. Aceton, Methylethylketon, Eisessig, ein Gemisch aus Eisessig und Aceton, DMSO, Methylenchlorid, Chloroform oder ein Gemisch aus Methylenchlorid und Chloroform, gelöst und die erhaltene Lösung mit Wasser, Methanol, Ethanol oder anderen Alkoholen als Fällungsmittel gemischt. Andere Lösungsmittel/Fällungsmittel sind denkbar (z.B. Fällung in Ether), aber aufgrund von Toxizitäten oder sicherheitstechnischen Aspekten großtechnisch nicht bevorzugt. Amorphe Polymere, welche sich nicht oder nur schwierig durch Extraktion reinigen lassen, werden häufig durch Umfällung gereinigt.

Bevorzugt ist ein Extraktionsverfahren. Dabei wird erhaltenes Rohpolymer mit einem Lösungsmittel extrahiert und anschließend getrocknet. Für die Extraktion werden die Rohpolymerisate üblicherweise zuvor zerkleinert, um eine ausreichende Diffusion des Lösungsmittels in den Festkörper zu gewährleisten. Für das Reinigungsverfahren sind organische Lösungsmittel und überkritische oder druckverflüssigte Gase geeignet, die das jeweilige Monomer, nicht jedoch das Polymer lösen. Bevorzugt werden organische Lösungsmittel aus der Gruppe der n-Alkane oder cyclo-Alkane (cyclo-Hexan oder n-Hexan bei Raumtemperatur) und Kohlendioxid, besonders bevorzugt wird überkritisches oder druckverflüssigtes Kohlendioxid verwendet.

Ein weiterer Gegenstand der vorliegenden Erfindung ist daher ein Verfahren zur Herstellung der erfindungsgemäßen Poly(etherester) vom AB- und ABA-Typ, umfassend die Schritte:
(a) Mischen und Aufschmelzen von Poly(ethylenglycol) mit einer oder zwei freien endständigen OH-Gruppen zusammen mit einem oder mehreren Monomeren bzw. Dimeren nach Formel III, 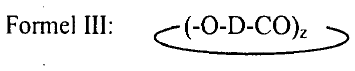 wobei D in jeder der Einheiten -O-D-CO unabhängig voneinander sein kann:

   -(CH(CH₃)-)ₓ

   oder

   -(CH₂-)ₓ

   oder

   -(CH₂-O-CH₂-CH₂-)

   oder

   -(CH₂-CH₂-CH₂-O-),

   x ist 1, 2, 3, 4 oder 5,
   und z eine ganze Zahl und mindestens 1 ist, bevorzugt 1 oder 2,
(b) Zugabe eines Metallkatalysators zur entstandenen homogenen Mischung aus (a),
(c) Polymerisieren der Mischung aus (b) bei einer Reaktionstemperatur, die oberhalb der Schmelztemperatur der jeweils eingesetzten Edukte liegt, während einer Reaktionszeit zwischen 24 Stunden und 5 Tagen,
(d) Reinigung der in Schritt (c) erhaltenen Rohpolymere durch Umfällung aus einem Lösungsmittel oder durch Extraktion und
(e) Zerkleinern der in Schritt (d) erhaltenen Polymere.

Vorzugsweise wird das in Schritt (a) eingesetzte Poly(ethylenglycol) zuvor getrocknet.
Die weiter bevorzugten Ausführungsformen können den voranstehend genannten Ausführungen entnommen werden.

### Herstellung der Implantate

Die resultierenden hochmolekularen Blockcopolymere lassen sich durch thermoplastische Formgebungsverfahren in leichter Weise zu chirurgischen Implantaten verarbeiten, welche gegenüber den aus dem Stand der Technik bekannten Implantaten das gewünschte beschleunigte Abbauverhalten und trotzdem eine hohe initiale Festigkeit aufweisen.

### Verwendung der erfindungsgemäßen Implantate

Die erfindungsgemäßen Implantate können beispielsweise für die Fixation von Hartgewebefrakturen (Osteosynthese), für die gesteuerte Geweberegeneration im Weichgewebe, für die Befestigung von Nahtfäden im Knochen (Nahtmaterialanker), als Wirbelsäulenimplantate zum Schutz der Bandscheibe (z.B. so genannte "spinal cages"), zum Verschließen und Verbinden von Blut- und anderen Gefäßen bei Gefäßrupturen (Anastomose), als Stents, zum Verfüllen von Kavitäten oder Löchern bei Gewebedefekten, beispielsweise im Dentalbereich oder bei Herzscheidewanddefekten oder auch zum Fixieren von Sehnen und Bändern im Knochen eingesetzt werden. Dafür werden die Blockcopolymere zu Formkörpern verarbeitet, deren Gestalt an den entsprechenden Verwendungszweck angepasst ist. So sind z.B. Schrauben, Dübel, Platten Muttern, Anker, Vliese, Folien, Membrane, Netze usw. zugänglich. Derartige Schrauben können beispielsweise in verschiedenen Dimensionierungen, mit verschiedenen Gewindeschneidungen, mit Rechts- oder Linksgewinde und mit verschiedenen Schraubenköpfen, beispielsweise Kreuz- oder Einfachschlitz, ausgestaltet werden. Weitere Anwendungsmöglichkeiten können dem Stand der Technik entnommen werden.

### Beispiele

Die nachfolgenden Beispiele dienen exemplarisch der Illustration und sind lediglich als Möglichkeiten zu verstehen, ohne die Erfindung in Ihrem Inhalt einzuschränken.

### 1. In Vitro Degradationsstudie

Die erfindungsgemäßen Polymere werden nach aus dem Stand der Technik bekannten Spritzgussverfahren zu Formkörpern verarbeitet (Prüfkörper). Diese werden in einem Drahtgewebe fixiert und kommen so in ein auf 37°C temperiertes Hydrolysebad. Dies wird mit einer Phosphatpufferlösung pH 7.4 befüllt, welche wöchentlich gewechselt wird. Zu festgelegten Prüfzeiten werden Proben entnommen. Der Abbau der Polymere wird über die Veränderung des Parameters inhärente Viskosität (i.V.) im zeitlichen Verlauf beobachtet (gemessen in einem Ubbelhode-Viskosimeter in Chloroform bei 25°C in 0.1 prozentiger Lösung).

### 1.1 Poly L-Lactid-Polyethylenglycol Di- oder Triblockcopolymere

Eingesetzt werden
- Polymere vom ABA-Typ mit L-Lactid als Baustein des A-Blocks und 1 und 5% Polyethylengycol 6000 (PEG 6000) als B-Block und
- Polymere vom AB-Typ mit L-Lactid als Baustein des A-Blocks und 1 und 5% Polyethylengycol 2000 (PEG 2000) als B-Block. Die Zahlen 2000 bzw. 6000 geben die Molmasse des PEG in Dalton an.

Der für die inhärente Viskosität ermittelte Wert zum Zeitpunkt 0 wird auf 100% normiert. Das entspricht dem Wert bevor der Prüfkörper in das Hydrolysebad eingetaucht worden ist. Um den Abbau zu bestimmen, werden die Messwerte in % bezugnehmend auf den Ausgangswert aufgetragen.

Als AB-Polymere werden eingesetzt:

| | |
|---|---|
| Probe 1: | L-Lactid-Polyethylenglycol mit einem Gewichtsanteil an Polyethylenglycol (PEG) von 1 % und einer Molmasse von 2000 Dalton. Die Länge des A-Blocks errechnet sich hierdurch auf 198000 Dalton. |
| | |
| Probe 2: | L-Lactid-Polyethylenglycol mit einem Gewichtsanteil an Polyethylenglycol (PEG) von 5% und einer Molmasse von 2000 Dalton. Die Länge des A-Blocks errechnet sich hierdurch auf 38000 Dalton. |

Als ABA-Polymere werden eingesetzt:

| | |
|---|---|
| Probe 3: | L-Lactid-Polyethylenglycol-L-Lactid mit einem Gewichtsanteil an Polyethylenglycol (PEG) von 1% und einer Molmasse von 6000 Dalton. Die Länge der A-Blöcke errechnet sich hierdurch auf jeweils 297000 Dalton. |
| | |
| Probe 4: | L-Lactid-Polyethylenglycol-L-Lactid mit einem Gewichtsanteil an Polyethylenglycol (PEG) von 5% und einer Molmasse von 6000 Dalton. Die Länge der A-Blöcke errechnet sich hierdurch auf jeweils 57000 Dalton. |

### Ergebnis:

Die nachfolgende Tabelle 1 gibt eine Übersicht über die Abbauraten der eingesetzten Polymere:

| | Poly L-Lactid (Vergleich) | | Probe 1 | | Probe 2 | | Probe 3 | | Probe 4 | |
|---|---|---|---|---|---|---|---|---|---|---|
| Dauer | i.V. | % | i.V. | % | i.V. | % | i.V. | % | i.V. | % |
| [Wochen] | [dl/g] | | [dl/g] | | [dl/g] | | [dl/g] | | [dl/g] | |
| 0 | 2.64 | 100 | 1.96 | 100 | 1.12 | 100 | 2.49 | 100 | 1.55 | 100 |
| 2 | 2.45 | 93 | 1.88 | 96 | 1.07 | 96 | 2.02 | 81 | 1.31 | 85 |
| 4 | n.b. | n.b. | 1.78 | 91 | 0.96 | 86 | 1.57 | 63 | 0.99 | 64 |
| 5 | 2.27 | 86 | n.b. | n.b. | n.b. | n.b. | n.b. | n.b. | n.b. | n.b. |
| 6 | n.b. | n.b. | 1.70 | 87 | 0.84 | 75 | 1.25 | 50 | 0.79 | 51 |
| 8 | n.b. | n.b. | 1.50 | 77 | 0.73 | 65 | 1.05 | 42 | 0.66 | 43 |
| 10 | n.b. | n.b. | 1.41 | 72 | 0.67 | 60 | 0.95 | 38 | 0.58 | 37 |
| 11 | 1.92 | 73 | n.b. | n.b. | n.b. | n.b. | n.b. | n.b. | n.b. | n.b. |
| 12 | n.b. | n.b. | 1.28 | 65 | 0.59 | 53 | 0.80 | 32 | 0.50 | 32 |
| 14 | n.b. | n.b. | 1.22 | 62 | 0.54 | 48 | 0.72 | 29 | 0.48 | 31 |
| 15 | 1.70 | 65 | n.b. | n.b. | n.b. | n.b. | n.b. | n.b. | n.b. | n.b. |
| 16 | n.b. | n.b. | 1.10 | 56 | 0.45 | 40 | 0.61 | 24 | 0.38 | 25 |
| 18 | n.b. | n.b. | 1.02 | 52 | 0.42 | 38 | 0.56 | 22 | 0.36 | 23 |
| 20 | 1.57 | 59 | n.b. | n.b. | n.b. | n.b. | n.b. | n.b. | n.b. | n.b. |

| | | | | | | | | | | |
|---|---|---|---|---|---|---|---|---|---|---|
| i.V. inhärente Viskosität (gemessen in einem Ubbelhode-Viskosimeter in Chloroform bei 25°C in 0.1 prozentiger Lösung) n.b. nicht bestimmt | | | | | | | | | | |

Bei den AB-Diblockcopolymeren ergeben sich nach 18 Wochen folgende Werte:
Probe 1: 52% des Ausgangswertes
Probe 2: 38% des Ausgangswertes

Bei den ABA-Triblockcopolymeren (Proben 3 und 4) führt der hydrolytische Abbau nach 18 Wochen, unabhängig ob 1 oder 5% PEG gewählt worden sind, zu einer i.V., die nur noch ca. 25% des Ausgangswertes ausmacht.

Zum Vergleich: der Wert für Poly L-Lactid liegt nach 20 Wochen im Bereich von knapp 60%.

### 1.2 Poly L-Lactid-co-D,L-Laetid-Polyethylenplycol Di- oder Triblockcopolvmere

Eingesetzt werden:
- Polymer vom ABA-Typ mit L-Lactid-co-D,L-Lactid als Baustein des A-Blocks und 5% Polyethylengycol 2000 (PEG 2000) als B-Block und
- Polymere vom AB-Typ mit L-Lactid-co-D,L-Lactid als Baustein des A-Blocks und 5% Polyethylengycol-MME 5000 (PEG-MME 5000) als B-Block. Die Zahlen 2000 bzw. 5000 geben die Molmasse des PEG bzw. PEG-MME in Dalton an.

Der für die inhärente Viskosität ermittelte Wert zum Zeitpunkt 0 wird auf 100% normiert. Das entspricht dem Wert bevor der Prüfkörper in das Hydrolysebad eingetaucht worden ist. Um den Abbau zu bestimmen, werden die Messwerte in % bezugnehmend auf den Ausgangswert aufgetragen.

Als ABA-Polymere werden eingesetzt:

| | |
|---|---|
| Probe 5: | L-Lactid-co-D,L-Lactid -Polyethylenglycol-L-Lactid-co-D,L-Lactid mit einem Gewichtsanteil an Polyethylenglycol (PEG) von 5% und einer Molmasse von 2000 Dalton. Die Länge der A-Blöcke errechnet sich hierdurch auf jeweils 19000 Dalton. |

Als AB-Polynere werden eingesetzt:

| | |
|---|---|
| Probe 6: | L-Lactid-co-D,L-Lactid -Polyethylenglycol mit einem Gewichtsanteil an Polyethylenglycol (PEG) von 5% und einer Molmasse von 5000 Dalton. Die Länge des A-Blocks errechnet sich hierdurch auf 95000 Dalton. |

### Ergebnis:

Bei dem ABA-Triblockcopolymer führt der hydrolytische Abbau nach 18 Wochen zu einer i.V., die nur noch ca. 30% des Ausgangswertes ausmacht.
Bei dem AB-Diblockcopolymer (Probe 6) ergibt sich nach 1.8 Wochen ein Viskositätswert von 9% des Ausgangswertes.

### 1.3 Poly L-Lactid-co-glycolid-Polyethylenglycol Di- oder Triblockcopolymere

Eingesetzt werden:
- Polymer vom ABA-Typ mit L-Lactid-co- Glycolid als Baustein des A-Blocks und 5% Polyethylengycol 2000 (PEG 2000) als B-Block und
- Polymere vom AB-Typ mit L-Lactid-co- Glycolid als Baustein des A-Blocks und 5% Polyethylengycol-MME 5000 (PEG-MME 5000) als B-Block. Die Zahlen 2000 bzw. 5000 geben die Molmasse des PEG bzw. PEG-MME in Dalton an.

Der für die inhärente Viskosität ermittelte Wert zum Zeitpunkt 0 wird auf 100% normiert. Das entspricht dem Wert bevor der Prüfkörper in das Hydrolysebad eingetaucht worden ist. Um den Abbau zu bestimmen, werden die Messwerte in % bezugnehmend auf den Ausgangswert aufgetragen.

Als ABA-Polymere werden eingesetzt:

| | |
|---|---|
| Probe 7: | L-Lactid-co- Glycolid -Polyethylenglycol-L-Lactid-co- Glycolid mit einem Gewichtsanteil an Polyethylenglycol (PEG) von 5% und einer Molmasse von 2000 Dalton. Die Länge der A-Blöcke errechnet sich hierdurch auf jeweils 19000 Dalton. |

Als AB-Polymere werden eingesetzt:

| | |
|---|---|
| Probe 8: | L-Lactid-co- Glycolid -Polyethylenglycol mit einem Gewichtsanteil an Polyethylenglycol (PEG) von 5% und einer Molmasse von 5000 Dalton. Die Länge des A-Blocks errechnet sich hierdurch auf 95000 Dalton. |

### Ergebnis:

Die nachfolgende Tabelle 2 gibt eine Übersicht über die Abbauraten der eingesetzten Polymere:

| | Probe 5 | | Probe 6 | | Probe 7 | | Probe 8 | |
|---|---|---|---|---|---|---|---|---|
| Dauer | i.V. | % | i.V. | % | i.V. | % | i.V. | % |
| [Wochen] | [dl/g] | | [dl/g] | | [dl/g] | | [dl/g] | |
| 0 | 0.86 | 100 | 1.49 | 100 | 0.89 | 100 | 1.24 | 100 |
| 2 | 0.83 | 97 | 1.35 | 91 | 0.81 | 91 | 0.93 | 75 |
| 4 | 0.79 | 92 | 1.12 | 75 | 0.67 | 75 | 0.62 | 50 |
| 6 | 0.72 | 84 | 0.85 | 57 | 0.50 | 56 | 0.46 | 37 |
| 8 | 0.62 | 72 | 0.55 | 37 | 0.38 | 43 | 0.29 | 23 |
| 10 | 0.52 | 60 | 0.42 | 28 | 0.25 | 28 | 0.20 | 16 |
| 12 | 0.41 | 48 | 0.29 | 19 | 0.18 | 20 | 0.17 | 14 |
| 14 | 0.34 | 40 | 0.21 | 14 | 0.14 | 16 | 0.13 | 10 |
| 16 | 0.28 | 33 | 0.18 | 12 | 0.11 | 12 | 0.10 | 8 |
| 18 | 0.25 | 29 | 0.14 | 9 | 0.10 | 11 | 0.13 | 10 |

| | | | | | | | | |
|---|---|---|---|---|---|---|---|---|
| i.V. inhärente Viskosität (gemessen in einem Ubbelhode-Viskosimeter in Chloroform bei 25°C in 0.1 prozentiger Lösung) | | | | | | | | |

Sowohl bei dem ABA-Triblockcopolymer als auch dem AB-Diblockcopolymer führt der hydrolytische Abbau nach 18 Wochen zu einer inhärenten Viskosität, die nur noch ca. 10% des Ausgangswertes ausmacht.

### 2. Mechanische Untersuchungen

Zur Bestimmung der Zugfestigkeit werden die im Folgenden aufgelisteten Prüfkörper gemäß ASTM D 638 hergestellt und vermessen:

| | |
|---|---|
| Probe 1: | L-Lactid-Pblyethylenglycol mit einem Gewichtsanteil an Polyethylenglycol (PEG) von 1% und einer Molmasse von 2000 Dalton. Die Länge des A-Blocks errechnet sich hierdurch auf 198000 Dalton. |
| | |
| Probe 2: | L-Lactid-Polyethylenglycol mit einem Gewichtsanteil an Polyethylenglycol (PEG) von 5% und einer Molmasse von 2000 Dalton. Die Länge des A-Blocks errechnet sich hierdurch auf 38000 Dalton. |
| | |
| Probe 3: | L-Lactid-Polyethylenglycol-L-Lactid mit einem Gewichtsanteil an Polyethylenglycol (PEG) von 1% und einer Molmasse von 6000 Dalton. Die Länge der A-Blöcke errechnet sich hierdurch auf jeweils 297000 Dalton. |
| | |
| Probe 4: | L-Lactid-Polyethylenglycol-L-Lactid mit einem Gewichtsanteil an Polyethylenglycol (PEG) von 5% und einer Molmasse von 6000 Dalton. Die Länge der A-Blöcke errechnet sich hierdurch auf jeweils 57000 Dalton. |
| Probe 5: | L-Lactid-Polyethylenglycol-L-Lactid mit einem Gewichtsanteil an Polyethylenglycol (PEG) von 15% und einer Molmasse von 6000 Dalton. |
| | |
| Probe 6: | L-Lactid-co-D,L-Lactid-Polyethylenglycol-L-Lactid-co-D,L-Lactid mit einem Gewichtsanteil an Polyethylenglycol (PEG) von 5% und einer Molmasse von 2000 Dalton und einem Verhältnis von L-Lactid zu D,L-Lactid = 70:30. |
| | |
| Probe 7: | L-Lactid-co-D,L-Lactid-Polyethylenglycol mit einem Gewichtsanteil an Polyethylenglycol (PEG) von 5% und einer Molmasse von 5000 Dalton und einem Verhältnis von L-Lactid zu D,L-Lactid = 70:30. |
| | |
| Probe 8: | L-Lactid-co-Glycolid-Polyethylenglycol-L-Lactid-co-Glycolid mit einem Gewichtsanteil.an Polyethylenglycol (PEG) von 5% und einer Molmasse von 2000 Dalton und einem Verhältnis von L-Lactid zu Glycolid =85:15. |
| | |
| Probe 9: | L-Lactid-co- Glycolid -Polyethylenglycol mit einem Gewichtsanteil an Polyethylenglycol (PEG) von 5% und einer Molmasse von 5000 Dalton und einem Verhältnis von L-Lactid zu Glycolid = 85:15. |
| | |
| Probe 10: | Poly(L-lactid-co-glycolid) mit einem Molverhältnis L-Lactid zu Glycolid von 85:15* |
| | |
| Probe 11: | Poly(L-lactid-co-DL-lactid) mit einem Molverhältnis L-Lactid zu DL-Lactid von 70:30* |

| | |
|---|---|
| *: Die Zugfestigkeit dieser Referenzmaterialien wurde nach DIN 53455 ermittelt, die entsprechenden Probekörper nach DIN 53452 hergestellt. | |

Die nachfolgende Tabelle 3 gibt eine Übersicht über die ermittelten Werte der Zugfestigkeit der eingesetzten Probenkörper:

| Name | i.V. [dl/g] | Zugfestigkeit [MPa] |
|---|---|---|
| Probe 1 | 1.96 | 84 |
| Probe 2 | 1.12 | 68 |
| Probe 3 | 2.49 | 85 |
| Probe 4 | 1.55 | 73 |
| Probe 5 | 0.6 | 23 |
| Probe 6 | 0.86 | 49 |
| Probe 7 | 1.5 | 55 |
| Probe 8 | 0.89 | 52 |
| Probe 9 | 1.7 | 65 |
| Probe 10 | 2.99 | 84 |
| Probe 11 | 3.06 | 74 |

| | | |
|---|---|---|
| i.V. inhärente Viskosität (gemessen in einem Ubbelhode-Viskosimeter in Chloroform bei 25°C in 0.1 prozentiger Lösung) | | |

### 3. Polymere

### 3.1 Polylactid-polyethylenlycol-polylactid-Triblockcopolymer

35 g PEG 6000 (Polyethylenglycol mit einem Molgewicht von 6000 Dalton, zwei endständige OH-Gruppen) werden bei 85°C und 50 mbar / 2 Stunden getrocknet. Es werden 3.5 kg L-Lactid zugegeben. Bei 112°C wird zu den geschmolzenen Edukten 965 mg Zinn(II)-2-ethylhexanoat zugegeben. Die Mischung wird bei 120°C 3 Tage lang in der Masse polymerisiert. Das entstandene Rohpolymer wird zerkleinert und mit den unten angegebenen Bedingungen extrahiert. Das Polymer hat eine i.V. von 2.63 dl/g und einen Restmonomergehalt an Lactid von unter 0.5%. Der Gehalt an PEG im Copolymeren beträgt 0.9% (¹H-NMR). Dies entspricht einer auf das PEG 6000 bezogenen Molmasse des A-Blocks von 338 000 g/mol je A-Block. Die Glasübergangstemperatur (Tg) (über DSC Messung, DSC 200 PC der Firma Netsch, Aufheizrate: 10°K/min) liegt bei 60.8°C.

### 3.2 Poly-D,L-lactid-co-L-lactid-polyethylenglycol-poly-D,L-lactid-co-L-lactid-Triblockcopolymer

175 g PEG 2000 (Polyethylenglycol mit einem Molgewicht von 2000 Dalton, zwei endständige OH-Gruppen) werden bei 85°C und 50 mbar / 2 Stunden getrocknet. Es werden 2520 g L-Lactid und 980 g D,L-Lactid zugegeben. Bei 112°C wird zu den geschmolzenen Edukten 1003 mg Zinn(II)-2-ethylhexanoat zugegeben. Die Mischung wird bei 120°C 3 Tage lang in der Masse polymerisiert. Das entstandene Rohpolymer wird zerkleinert und mit den unten angegebenen Bedingungen extrahiert. Das Polymer hat eine i.V. von 0.86 dl/g und einen Restmonomergehalt an Lactid von unter 0.5%. Der Gehalt an PEG im Copolymeren beträgt 4.8% (¹H-NMR).

### 3.3 Poly-L-lactid-co-glycolid-polyethylenglycol-Diblockcopolymer

125 g PEG-MME 2000 (Polyethylenglycol mit einem Molgewicht von 2000 Dalton, einer endständigen OH-Gruppe und einer endständigen Methoxygruppe) werden bei 85°C und 50 mbar / 2 Stunden getrocknet. Es werden 2135.2 g L-Lactid und 364.8 g Glycolid zugegeben. Bei 112°C wird zu den geschmolzenen Edukten 717 mg Zinn(II)-2-ethylhexanoat zugegeben. Die Mischung wird bei 150°C 3 Tage lang in der Masse polymerisiert. Das entstandene Rohpolymer wird zerkleinert und extrahiert. Das Polymer hat eine i.V. von 1.7 dl/g und einen Restmonomergehalt von unter 0.5%. Der Gehalt an PEG im Copolymeren beträgt 5.3% (¹H-NMR).

### 3.4 Poly-D,L-lactid-co-glycolid-polyethylenglycol-1-Diblockcopolymer

236.7 g PEG-MME 5000 (Polyethylenglycol mit einem Molgewicht von 5000 Dalton, einer endständigen OH-Gruppe und einer endständigen Methoxygruppe) werden bei 85°C und 50 mbar / 2 Stunden getrocknet. Es werden 2537 g D,L-Lactid und 1936 g Glycolid zugegeben. Bei 112°C wird zu den geschmolzenen Edukten 1293 mg Zinn(II)-2-ethylhexanoat zugegeben. Die Mischung wird bei 150°C 3 Tage lang in der Masse polymerisiert. Das entstandene Rohpolymer wird durch Lösen in Aceton und Fällung in Wasser gereinigt und anschließend getrocknet. Das Polymer hat eine i.V. von 1.1 dl/g und einen Restmonomergehalt von unter 0.5%. Der Gehalt an PEG im Copolymeren beträgt 4.9% (¹H-NMR).

### 3.5 Poly-L-lactid-polyethylenglycol-MME-Diblockcopolymer

35 g PEG-MME 2000 (Polyethylenglycol mit einem Molgewicht von 2000 Dalton, einer endständigen OH-Gruppe und einer endständigen Methoxygruppe) werden bei 85°C und 50 mbar /2 Stunden getrocknet. Es werden 3500 g L-Lactid zugegeben. Bei 112°C wird zu den geschmolzenen Edukten 965 mg Zinn(II)-2-ethylhexanoat zugegeben. Die Mischung wird bei 120°C 5-7 Tage lang in der Masse polymerisiert. Das entstandene Rohpolymer wird zerkleinert und extrahiert. Das Polymer hat eine i.V. von 1.91 dl/g und einen Restmonomergehalt von unter 0.5%. Der Gehalt an PEG im Copolymeren beträgt 0.94% (¹H-NMR).

### 3.6 Poly-L-lactid-co-D,L-lactid-polyethylenglycol-MME-Diblockcopolymer

175 g PEG-MME 5000 (Polyethylenglycol mit einem Molgewicht von 5000 Dalton, einer endständigen OH-Gruppen und einer endständigen Methoxygruppe) werden bei 85°C und 50 mbar / 2 Stunden getrocknet. Es werden 2520.0 g L-Lactid und 980 g D, L-Lactid zugegeben. Bei 112°C wird zu den geschmolzenen Edukten 1003 mg Zinn(II) 2-ethylhexanoat zugegeben. Die Mischung wird bei 120°C 3 Tage lang in der Masse polymerisiert. Das entstandene Rohpolymer wird zerkleinert und extrahiert Das Polymer hat eine i.V. von 1.55 dl/g und einen Restmonomergehalt von unter 0.5%. Der Gehalt an PEG im Copolymeren beträgt 4.84% (¹H-NMR).

### 3.7 Poly-L-lactid-co-glycolid-polyethylenglycol-poly-L-lactid-co-glycolid-Triblockcopolymer

65 g PEG- 6000 (Polyethylenglycol mit einem Molgewicht von 6000 Dalton, zwei endständigen OH-Gruppen) werden bei 85°C und 50 mbar / 2 Stunden getrocknet. Es werden 5496.1 g L-Lactid und 938.9 g Glycolid zugegeben. Bei 112°C wird zu den geschmolzenen Edukten 1775 mg Zinn(II) 2-ethylhexanoat zugegeben. Die Mischung wird bei 150°C 3 Tage lang in der Masse polymerisiert. Das entstandene Rohpolymer wird zerkleinert und extrahiert. Das Polymer hat eine i.V. von 2.7 dl/g und einen Restmonomergehalt von unter 0.5%. Der Gehalt an PEG im Copolymeren beträgt 1.0% (¹H-NMR).

### 3.8 Poly-D,L-lactid-co-glycolid-polyethylenglycol-poly-D,L-lactid-co-glycolylid-Triblockcopolymer

499.5 g PEG 6000 (Polyethylenglycol mit einem Molgewicht von 6000 Dalton, zwei endständigen OH-Gruppen) werden bei 85°C und 50 mbar / 2 Stunden getrocknet. Es werden 2537.0 g D,L-Lactid und 1963.0 g Glycolid zugegeben. Bei 112°C wird zu den geschmolzenen Edukten 1365 mg Zinn(II)-2-ethylhexanoat zugegeben. Die Mischung wird bei 150°C 3 Tage lang in der Masse polymerisiert. Das entstandene Rohpolymer wird durch lösen in Aceton und Fällung in Wasser gereinigt und anschließend getrocknet. Das Polymer hat eine i.V. von 0.75 dl/g und einen Restmonomergehalt von unter 0.5%. Der Gehalt an PEG im Copolymeren beträgt 10.05% (¹H-NMR).

### 4. Reinigung

Die gemahlenen Produkte aus Beispielen 3.1 bis 3.3 und 3.5 bis 3.7 werden in eine 16 L Extraktionskartusche eingefüllt. Die Kartusche wird verschlossen und der Inhalt anschließend mit druckverflüssigtem Kohlendioxid extrahiert.

### Extraktionsbedingungen:

| | |
|---|---|
| Zeit/Druck: | 1 h bei 90 bar, anschließend 4 h bei 300 bar |
| Temperatur: | ≤ 10°C |
| Kohlendioxid-Strömung: | ca.120kg/h |

Dieses Reinigungsbeispiel lässt sich analog für andere Polymere durchführen.

## Patentansprüche

1. Blockcopolymer vom AB- oder ABA-Typ mit A einem Polyesterblock und B einem Polyetherblock, worin der AB-Typ durch die Formel I repräsentiert wird:
E-(O-D-CO-)ₙ-(O-CH₂-CH₂-)ₘ-O-F (Formel I)
wobei
die Struktureinheit E-(-O-D-CO-)ₙ- den Block A bildet,
-(O-CH₂-CH₂-)ₘ- den Block B bildet,
D für jede der n Einheiten unabhängig voneinander sein kann:
-(CH(CH₃)-)ₓ
oder
-(CH₂-)ₓ
oder
-(CH₂-O-CH₂-CH₂-)
oder
-(CH₂-CH₂-CH₂-O-),
x 1, 2, 3, 4 oder 5 ist,
E und F unabhängig voneinander H, Methyl oder Ethyl sind,
n und m statistisch gemittelt und unabhängig voneinander eine integere Zahl größer 1 sind
und der B-Block einen Anteil zwischen 0.1 und 4 Gew.% ausmacht, und worin der ABA-Typ durch die Formel II repräsentiert wird:
E(-O-D-CO)ₙ-(O-CH₂-CH₂-)ₘ-O-(CO-D-O-)ₙ-E, (Formel II)
wobei
die Struktureinheiten E-(-O-D-CO-)ₙ- und E-(-O-D-CO-)_{n'}- die Blöcke A bilden,
-(O-CH₂-CH₂-)ₘ- den Block B bildet,
D für jede der n oder n' Einheiten unabhängig voneinander sein kann:
-(CH(CH₃)-)ₓ
oder
-(CH₂-)ₓ
oder
-(CH₂-O-CH₂-CH₂-)
oder
-(CH₂-CH₂-CH₂-O-),
x 1, 2, 3, 4 oder 5 ist,
E H, Methyl oder Ethyl ist,
n, n' und m statistisch gemittelt und unabhängig voneinander eine integere Zahl größer 1 sind,
und der B-Block einen Anteil zwischen 0.1 und 4 Gew.% ausmacht, **dadurch gekennzeichnet, dass** A aufgebaut ist aus einem Gemisch von Monomerbausteinen ausgewählt aus der Gruppe a) D- und L-Lactid, b) L-Lactid und Glycolid, c) D,L-Lactid und Glycolid, d) L-Lactid und epsilon-Caprolacton, e) L-Lactid und Dioxanon, f) L-Lactid und Trimethylencarbonat.

2. Blockcopolymer gemäß Anspruch 1 **dadurch gekennzeichnet, dass** der Gewichtsanteil des B-Blocks zwischen 1 und 3 Gew.% liegt.

3. Blockcopolymer vom AB- oder ABA-Typ, **dadurch gekennzeichnet, dass** es durch die Formel I oder durch die Formel II gemäß Anspruch 1 repräsentiert wird und dass der A-Block aus statistisch verteilten Einheiten des L-Lactids und des DL-Lactids aufgebaut ist.

4. Blockcopolymer nach Anspruch 3, **dadurch gekennzeichnet, dass** der molare Anteil des L-Lactids im A-Block zwischen 60 und 90% beträgt.

5. Blockcopolymer vom AB- oder ABA-Typ, **dadurch gekennzeichnet, dass** es durch die Formel I oder durch die Formel II gemäß Anspruch 1 repräsentiert wird und dass der A-Block aus statistisch verteilten Einheiten des L-Lactids und des Glycolids mit einem molaren Anteil des L-Lactids zwischen 85 und 99% besteht.

6. Blockcopolymer nach Anspruch 5, **dadurch gekennzeichnet, dass** der molare Anteil des L-Lactids im A-Block zwischen 87 und 95% beträgt.

7. Blockcopolymer vom AB- oder ABA-Typ, **dadurch gekennzeichnet, dass** es durch die Formel I oder durch die Formel II gemäß Anspruch 1 repräsentiert wird und dass der A-Block aus statistisch verteilten Einheiten des DL-Lactids und des Glycolids besteht und die inhärente Viskosität einen Wert von größer 0.8 dl/g aufweist.

8. Blockcopolymer nach Anspruch 7, **dadurch gekennzeichnet, dass** der molare Anteil des DL-Lactids im A-Block zwischen 50 und 80% beträgt.

9. Blockcopolymer nach Anspruch 1, **dadurch gekennzeichnet, dass** der A-Block aus statistisch verteilten Einheiten des L-Lactids oder DL-Lactids und des Dioxanons besteht.

10. Blockcopolymer nach Anspruch 1, **dadurch gekennzeichnet, dass** der A-Block aus statistisch verteilten Einheiten des L-Lactids oder DL-Lactids und des Trimethylencarbonats besteht.

11. Blockcopolymer nach einem der Ansprüche 1 bis 10, **dadurch gekennzeichnet, dass** die zahlenmittlere Blocklänge des B-Blocks zwischen 500 und 10000 Dalton liegt.

12. Blockcopolymer nach Anspruch 11, **dadurch gekennzeichnet, dass** die zahlenmittlere Blocklänge des B-Blocks zwischen 600 und 8000 Dalton liegt.

13. Blockcopolymer nach Anspruch 12, **dadurch gekennzeichnet, dass** die zahlenmittlere Blocklänge des B-Blocks zwischen 1000 und 8000 Dalton liegt.

14. Blockcopolymer nach einem der Ansprüche 1 bis 13, **dadurch gekennzeichnet, dass** die inhärente Viskosität zwischen 0.1 und 6 dl/g liegt.

15. Blockcopolymer nach Anspruch 14, **dadurch gekennzeichnet, dass** die inhärente Viskosität zwischen 0.5 und 5 dl/g liegt.

16. Blockcopolymer nach Anspruch 15, **dadurch gekennzeichnet, dass** die inhärente Viskosität zwischen 0.6 und 3 dl/g liegt.

17. Blockcopolymer nach Anspruch 16, **dadurch gekennzeichnet, dass** die inhärente Viskosität zwischen 0.7 und 2.75 dl/g liegt.

18. Verfahren zur Herstellung von Poly(etherestern) vom AB- und ABA-Typ gemäß einem der Ansprüche 1 bis 17, umfassend die Schritte:
(a) Mischen und Aufschmelzen von Poly(ethylenglycol) mit einer oder zwei freien endständigen OH-Gruppen zusammen mit einem oder mehreren Monomeren bzw. Dimeren nach Formel III, 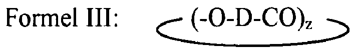 wobei D in jeder der Einheiten -O-D-CO unabhängig voneinander sein kann:
-(CH(CH₃)-)ₓ
oder
-(CH₂-)ₓ
oder
-(CH₂-O-CH₂-CH₂-)
oder
-(CH₂-CH₂-CH₂-O-),
x ist 1, 2, 3, 4 oder 5,
und z eine ganze Zahl und mindestens 1 ist, bevorzugt 1 oder 2,
(b) Zugabe eines Metallkatalysators zur entstandenen homogenen Mischung aus (a),
(c) Polymerisieren der Mischung aus (b) bei einer Reaktionstemperatur, die oberhalb der Schmelztemperatur der jeweils eingesetzten Edukte liegt, während einer Reaktionszeit zwischen 24 Stunden und 5 Tagen,
(d) Reinigung der in Schritt (c) erhaltenen Rohpolymere durch Umfällung aus einem Lösungsmittel oder durch Extraktion und
(e) Zerkleinern der in Schritt (d) erhaltenen Polymere.

19. Verfahren gemäß Anspruch 18, **dadurch gekennzeichnet, dass** das in Schritt (a) eingesetzte Poly(ethylenglycol) zuvor getrocknet wird.

20. Verfahren gemäß Anspruch 18, **dadurch gekennzeichnet, dass** als Metallkatalysator in Schritt (b) Zinn(II)chlorid oder Zinn(II)ethylhexanoat in einer Konzentration zwischen 30 und 200 ppm verwendet wird.

21. Verfahren gemäß Anspruch 18, **dadurch gekennzeichnet, dass** als Metallkatalysator in Schritt (b) Zinn(11)chlorid oder Zinn(II)ethylhexanoat in einer Konzentration zwischen 50 und 100 ppm verwendet wird.

22. Verfahren gemäß Anspruch 18, **dadurch gekennzeichnet, dass** das Polymerisieren in Schritt (c) in einem Temperaturbereich zwischen 100 und 160°C erfolgt.

23. Verfahren gemäß Anspruch 18, **dadurch gekennzeichnet, dass** das Polymerisieren in Schritt (c) in einem Temperaturbereich zwischen 100 und 140°C erfolgt.

24. Verfahren gemäß Anspruch 18, **dadurch gekennzeichnet, dass** das Polymerisieren in Schritt (c) in einem Temperaturbereich zwischen 110 und 130°C erfolgt.

25. Verfahren gemäß Anspruch 18, **dadurch gekennzeichnet, dass** das Polymerisieren in Schritt (c) während einer Reaktionszeit zwischen 2 und 5 Tagen erfolgt.

26. Verfahren gemäß Anspruch 18, **dadurch gekennzeichnet, dass** die Reinigung in Schritt (d) durch Extraktion erfolgt, wobei das in Schritt (c) erhaltene Rohpolymer mit einem Lösungsmittel aus der Gruppe der n- oder cyclo-Alkane oder mit Kohlendioxid, extrahiert wird.

27. Verfahren gemäß Anspruch 18, **dadurch gekennzeichnet, dass** die Reinigung in Schritt (d) durch Extraktion erfolgt, wobei das in Schritt (c) erhaltene Rohpolymer mit Kohlendioxid, extrahiert wird.

28. Implantat enthaltend ein Blockcopolymer gemäß einem der Ansprüche 1 bis 17.

29. Implantat nach Anspruch 28, **dadurch gekennzeichnet, dass** das Implantat ausschließlich aus einem Werkstoff gefertigt ist.

30. Implantat nach einem der Ansprüche 28 bis 29 zur Fixation von Hartgewebefrakturen, als Nahtmaterialanker, als Wirbelsäulenimplantat, zum Verschließen und Verbinden von Blut- und anderen Gefäßen, als Stent, zum Verfüllen von Kavitäten oder Löchern bei Gewebedefekten.

31. Implantat nach einem der Ansprüche 28 bis 30 als Stent, **dadurch gekennzeichnet, dass** dieser einen Wirkstoff enthält, der ausgewählt ist aus der Gruppe der Zytostatica.

32. Implantat nach einem der Ansprüche 28 bis 30 in Form einer Schraube, eines Dübels, einer Platte, einer Mutter, einem Anker, einem Vlies, einer Folie, einer Membran, oder eines Netzes.

33. Verfahren zur Herstellung eines Implantats nach einem der Ansprüche 28 bis 32, umfassend die Schritte:
(a) Trocknung eines Blockcopolymeren der Formel I oder II gemäß einem der Ansprüche 1 bis 17,
(b) Überführung des Blockcopolymeren in den Schmelzzustand in einer Spritzgussmaschine,
(c) Einspritzen der Polymerschmelze in eine Form der gewünschten Dimensionierung,
(d) Abkühlen der Polymerschmelze in der Spritzgussform und
(e) Isolierung des Formkörpers aus der Spritzgussform.

## Claims

1. Block copolymer of the AB or ABA type, with A being a polyester block and B being a polyether block, wherein the AB type is represented by the formula I:
E-(O-D-CO-)ₙ-(O-CH₂-CH₂-)ₘ-O-F (formula I)
where
the structural unit E-(-O-D-CO-)ₙ- forms the block A,
-(O-CH₂-CH₂-)ₘ- forms the block B,
D may be, for each of the n units independently of one another:
- (CH(CH₃)-)ₓ
or
-(CH₂-)ₓ
or
- (CH₂-O-CH₂-CH₂-)
or
-(CH₂-CH₂-CH₂-O-),
x is 1, 2, 3, 4 or 5,
E and F independently of one another are H, methyl or ethyl,
n and m, statistically averaged and independently of one another, are an integer greater than 1
and the B block makes up a proportion of between 0.1 and 4 wt.%, and in which the ABA type is represented by the formula II:
E(-O-D-CO)ₙ-(O-CH₂-CH₂-)ₘ-O-(CO-D-O-)_{n'}-E (formula II),
where
the structural units E-(-O-D-CO-)ₙ- and E-(-O-D-CO-)_{n'}- form the blocks A,
-(O-CH₂-CH₂-)ₘ- forms the block B,
D may be, for each of the n or n' units independently of one another:
-(CH(CH₃)-)ₓ
or
-(CH₂-)ₓ
-(CH₂-O-CH₂-CH₂-)
-(CH₂-CH₂-CH₂-O-),
x is 1, 2, 3, 4 or 5,
E is H, methyl or ethyl,
n, n' and m, statistically averaged and independently of one another, are an integer greater than 1,
and the B block makes up a proportion of between 0.1 and 4 wt.%, **characterized in that** A is synthesized from a mixture of monomer components selected from the group consisting of a) D- and L-lactide, b) L-lactide and glycolide, c) D,L-lactide and glycolide, d) L-lactide and epsilon-caprolactone, e) L-lactide and dioxanone, and f) L-lactide and trimethylene carbonate.

2. Block copolymer according to Claim 1, **characterized in that** the proportion by weight of the B block is between 1 and 3 wt.%.

3. Block copolymer of the AB or ABA type, **characterized in that** it is represented by the formula I or by the formula II according to Claim 1 and **in that** the A block is synthesized from statistically distributed units of L-lactide and of DL-lactide.

4. Block copolymer according to Claim 3, **characterized in that** the molar proportion of L-lactide in the A block is between 60 and 90%.

5. Block copolymer of the AB or ABA type, **characterized in that** it is represented by the formula I or by the formula II according to Claim 1 and **in that** the A block consists of statistically distributed units of L-lactide and of glycolide, with a molar proportion of the L-lactide of between 85 and 99%.

6. Block copolymer according to Claim 5, **characterized in that** the molar proportion of the L-lactide in the A block is between 87 and 95%.

7. Block copolymer of the AB or ABA type, **characterized in that** it is represented by the formula I or by the formula II according to Claim 1 and **in that** the A block consists of statistically distributed units of DL-lactide and of glycolide and the intrinsic viscosity has a value of more than 0.8 dl/g.

8. Block copolymer according to Claim 7, **characterized in that** the molar proportion of the DL-lactide in the A block is between 50 and 80%.

9. Block copolymer according to Claim 1, **characterized in that** the A block consists of statistically distributed units of L-lactide or DL-lactide and of dioxanone.

10. Block copolymer according to Claim 1, **characterized in that** the A block consists of statistically distributed units of L-lactide or DL-lactide and of trimethylene carbonate.

11. Block copolymer according to any of Claims 1 to 10, **characterized in that** the numerically average block length of the B block is between 500 and 10 000 Dalton.

12. Block copolymer according to Claim 11, **characterized in that** the numerically average block length of the B block is between 600 and 8000 Dalton.

13. Block copolymer according to Claim 12, **characterized in that** the numerically average block length of the B block is between 1000 and 8000 Dalton.

14. Block copolymer according to any of Claims 1 to 13, **characterized in that** the intrinsic viscosity is between 0.1 and 6 dl/g.

15. Block copolymer according to Claim 14, **characterized in that** the intrinsic viscosity is between 0.5 and 5 dl/g.

16. Block copolymer according to Claim 15, **characterized in that** the intrinsic viscosity is between 0.6 and 3 dl/g.

17. Block copolymer according to Claim 16, **characterized in that** the intrinsic viscosity is between 0.7 and 2.75 dl/g.

18. Process for preparing poly(etheresters) of the AB and ABA type according to any of Claims 1 to 17, comprising the steps of:
(a) mixing and melting poly(ethylene glycol) with one or two free terminal OH groups together with one or more monomers and/or dimers according to formula III, 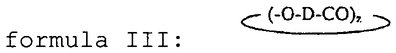 where D in each of the units -O-D-CO independently of one another may be:
- (CH (CH₃) -)
or
- (CH₂-)ₓ
or
- (CH₂-O-CH₂-CH₂-)
or
- (CH₂-CH₂-CH₂-O-),
x is 1, 2, 3, 4 or 5,
and z is an integer and is at least 1, preferably 1 or 2,
(b) adding a metal catalyst to the homogeneous mixture obtained from (a),
(c) polymerizing the mixture from (b) at a reaction temperature which is above the melting temperature of each of the reactants used, for a reaction time of between 24 hours and 5 days,
(d) purifying the crude polymers obtained in step (c) by precipitation from a solvent or by extraction and
(e) comminuting the polymers obtained in step (d).

19. Process according to Claim 18, **characterized in that** the poly(ethylene glycol) used in step (a) is dried beforehand.

20. Process according to Claim 18, **characterized in that**, as metal catalyst in step (b), tin(II) chloride or tin(II) ethylhexanoate in a concentration of between 30 and 200 ppm is used.

21. Process according to Claim 18, **characterized in that**, as metal catalyst in step (b), tin(II) chloride or tin(II) ethylhexanoate in a concentration of between 50 and 100 ppm is used.

22. Process according to Claim 18, **characterized in that** the polymerizing in step (c) takes place in a temperature range of between 100 and 160°C.

23. Process according to Claim 18, **characterized in that** the polymerizing in step (c) takes place in a temperature range of between 100 and 140°C.

24. Process according to Claim 18, **characterized in that** the polymerizing in step (c) takes place in a temperature range of between 110 and 130°C.

25. Process according to Claim 18, **characterized in that** the polymerizing in step (c) takes place for a reaction time of between 2 and 5 days.

26. Process according to Claim 18, **characterized in that** the purifying in step (d) takes place by extraction, the crude polymer obtained in step (c) being extracted with a solvent from the group of the n-alkanes or cycloalkanes or with carbon dioxide.

27. Process according to Claim 18, **characterized in that** the purifying in step (d) takes place by extraction, the crude polymer obtained in step (c) being extracted with carbon dioxide.

28. Implant comprising a block copolymer according to any of Claims 1 to 17.

29. Implant according to Claim 28, **characterized in that** the implant is fabricated exclusively from one material.

30. Implant according to either of Claims 28 and 29 for fixing hard tissue fractures, as suture material anchor, as spinal implant, for closing and joining blood vessels and other vessels, as a stent, for filling cavities or holes in the case of tissue defects.

31. Implant according to any of Claims 28 to 30 as a stent, **characterized in that** it comprises an active ingredient selected from the group of the cytostatics.

32. Implant according to any of Claims 28 to 30 in the form of a screw, pin, plate, nut, anchor, fleece, film, membrane or mesh.

33. Process for producing an implant according to any of Claims 28 to 32, comprising the steps of:
(a) drying a block copolymer of the formula I or II according to any of Claims 1 to 17,
(b) converting the block copolymer into the melt state in an injection moulding machine,
(c) injecting the polymer melt into a mould of the desired dimensions,
(d) cooling the polymer melt in the injection mould and
(e) isolating the moulding from the injection mould.

## Revendications

1. Copolymère à blocs de type AB ou ABA avec A représentant un bloc polyester et B un bloc polyéther, le type AB étant représenté par la formule I
E-(O-D-CO-)ₙ-(O-CH₂-CH₂-)ₘ-O-F (Formule I)
où
l'unité de structure E-(-O-D-CO-)ₙ- forme le bloc A,
-(O-CH₂-CH₂-)ₘ- forme le bloc B,
D peut représenter, pour chacune des n unités, indépendamment l'un de l'autre
- (CH (CH₃)-)ₓ
ou
- (CH₂-)ₓ
ou
- (CH₂-O-CH₂-CH₂-)
ou
- (CH₂-CH₂-CH₂-O-),
x vaut 1, 2, 3, 4 ou 5,
E et F représentent, indépendamment l'un de l'autre, H, méthyle ou éthyle
n et m valent, en moyenne statistique et indépendamment l'un de l'autre, un nombre entier supérieur à 1
et le bloc B représente une proportion entre 0,1 et 4% en poids, et le type ABA étant représenté par la formule II :
E(-O-D-CO)ₙ-(O-CH₂-CH₂-)ₘ-O-(CO-D-O-)_{n'}-E (Formule II),
où
les unités de structure E-(-O-D-CO-)ₙ- et E-(-O-D-CO-)_{n'}- forment les blocs A,
-(O-CH₂-CH₂-)ₘ- forme le bloc B,
D peut représenter, pour chacune des n ou n' unités, indépendamment l'un de l'autre :
-(CH(CH₃)-)ₓ
ou
-(CH₂-)ₓ
ou
-(CH₂-O-CH₂-CH₂-)
ou
-(CH₂-CH₂-CH₂-O-),
x vaut 1, 2, 3, 4 ou 5,
E représente H, méthyle ou éthyle,
n, n' et m valent, en moyenne statistique et indépendamment l'un de l'autre, un nombre entier supérieur à 1
et le bloc B représente une proportion entre 0,1 et 4% en poids, **caractérisé en ce que** A est formé à partir d'un mélange d'éléments monomères choisis dans le groupe formé par a) le D-lactide et L-lactide, b) le L-lactide et le glycolide, c) le D,L-lactide et le glycolide, d) le L-lactide et l'epsilon-caprolactone, e) le L-lactide et la dioxanone, f) le L-lactide et le triméthylènecarbonate.

2. Copolymère à blocs selon la revendication 1, **caractérisé en ce que** la proportion pondérale du bloc B est située entre 1 et 3% en poids.

3. Copolymère à blocs du type AB ou ABA, **caractérisé en ce qu'**il est représenté par la formule I ou par la formule II selon la revendication 1 et **en ce que** le bloc A est constitué par des unités statistiquement réparties de L-lactide et de DL-lactide.

4. Copolymère à blocs selon la revendication 3, **caractérisé en ce que** la proportion molaire du L-lactide dans le bloc A est située entre 60 et 90%.

5. Copolymère à blocs du type AB ou ABA, **caractérisé en ce qu'**il est représenté par la formule I ou par la formule II selon la revendication 1 et **en ce que** le bloc A est constitué par des unités statistiquement réparties de L-lactide et de glycolide avec une proportion molaire du L-lactide entre 85 et 99%.

6. Copolymère à blocs selon la revendication 5, **caractérisé en ce que** la proportion molaire du L-lactide dans le bloc A est située entre 87 et 95%.

7. Copolymère à blocs du type AB ou ABA, **caractérisé en ce qu'**il est représenté par la formule I ou par la formule II selon la revendication 1 et **en ce que** le bloc A est constitué par des unités statistiquement réparties de DL-lactide et de glycolide et la viscosité inhérente présente une valeur supérieure à 0,8 dl/g.

8. Copolymère à blocs selon la revendication 7, **caractérisé en ce que** la proportion molaire du DL-lactide dans le bloc A est située entre 50 et 80%.

9. Copolymère à blocs selon la revendication 1, **caractérisé en ce que** le bloc A est constitué par des unités statistiquement réparties de L-lactide ou de DL-lactide et de dioxanone.

10. Copolymère à blocs selon la revendication 1, **caractérisé en ce que** le bloc A est constitué par des unités statistiquement réparties de L-lactide ou de DL-lactide et de triméthylènecarbonate.

11. Copolymère à blocs selon l'une quelconque des revendications 1 à 10, **caractérisé en ce que** la longueur numérique moyenne du bloc B est située entre 500 et 10 000 Daltons.

12. Copolymère à blocs selon la revendication 11, **caractérisé en ce que** la longueur numérique moyenne du bloc B est située entre 600 et 8000 Daltons.

13. Copolymère à blocs selon la revendication 12, **caractérisé en ce que** la longueur numérique moyenne du bloc B est située entre 1000 et 8000 Daltons.

14. Copolymère à blocs selon l'une quelconque des revendications 1 à 13, **caractérisé en ce que** la viscosité inhérente est située entre 0,1 et 6 dl/g.

15. Copolymère à blocs selon la revendication 14, **caractérisé en ce que** la viscosité inhérente est située entre 0,5 et 5 dl/g.

16. Copolymère à blocs selon la revendication 15, **caractérisé en ce que** la viscosité inhérente est située entre 0,6 et 3 dl/g.

17. Copolymère à blocs selon la revendication 16, **caractérisé en ce que** la viscosité inhérente est située entre 0,7 et 2,75 dl/g.

18. Procédé pour la préparation de poly(éther-esters) de type AB et ABA selon l'une quelconque des revendications 1 à 17, comprenant les étapes :
(a) mélange et fusion de poly(éthylèneglycol) présentant un ou deux groupes OH libres en position d'extrémité ensemble avec un ou plusieurs monomères ou, selon le cas, dimères selon la formule III, 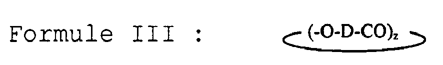 où D peut représenter, dans chacune des unités -O-D-CO, indépendamment l'un de l'autre :
- (CH(CH₃)-)ₓ
ou
- (CH₂-)ₓ
- (CH₂-O-CH₂-CH₂-)
ou
- (CH₂-CH₂-CH₂-O-),
x vaut 1, 2, 3, 4 ou 5,
et z vaut un nombre entier et au moins 1, de préférence 1 ou 2,
(b) addition d'un catalyseur métallique au mélange homogène formé de (a),
(c) polymérisation du mélange de (b) à une température de réaction qui est supérieure à la température de fusion des produits de départ à chaque fois utilisés, pendant un temps de réaction entre 24 heures et 5 jours,
(d) purification des polymères bruts obtenus dans l'étape (c) par reprécipitation à partir d'un solvant ou par extraction et
(e) broyage des polymères obtenus dans l'étape (d).

19. Procédé selon la revendication 18, **caractérisé en ce que** le poly(éthylèneglycol) utilisé dans l'étape (a) est séché au préalable.

20. Procédé selon la revendication 18, **caractérisé en ce qu'**on utilise comme catalyseur métallique dans l'étape (b) du chlorure d'étain (II) ou de l'éthylhexanoate d'étain (II) en une concentration entre 30 et 200 ppm.

21. Procédé selon la revendication 18, **caractérisé en ce qu'**on utilise comme catalyseur métallique dans l'étape (b) du chlorure d'étain (II) ou de l'éthylhexanoate d'étain (II) en une concentration entre 50 et 100 ppm.

22. Procédé selon la revendication 18, **caractérisé en ce que** la polymérisation dans l'étape (c) est réalisée dans une plage de température entre 100 et 160°C.

23. Procédé selon la revendication 18, **caractérisé en ce que** la polymérisation dans l'étape (c) est réalisée dans une plage de température entre 100 et 140°C.

24. Procédé selon la revendication 18, **caractérisé en ce que** la polymérisation dans l'étape (c) est réalisée dans une plage de température entre 110 et 130°C.

25. Procédé selon la revendication 18, **caractérisé en ce que** la polymérisation dans l'étape (c) est réalisée pendant un temps de réaction entre 2 et 5 jours.

26. Procédé selon la revendication 18, **caractérisé en ce que** la purification dans l'étape (d) est réalisée par extraction, le polymère brut obtenu dans l'étape (c) étant extrait avec un solvant du groupe des n-alcanes ou des cycloalcanes ou avec du dioxyde de carbone.

27. Procédé selon la revendication 18, **caractérisé en ce que** la purification dans l'étape (d) est réalisée par extraction, le polymère brut obtenu dans l'étape (c) étant extrait avec du dioxyde de carbone.

28. Implant contenant un copolymère à blocs selon l'une quelconque des revendications 1 à 17.

29. Implant selon la revendication 28, **caractérisé en ce que** l'implant est réalisé exclusivement à partir d'un matériau.

30. Implant selon l'une quelconque des revendications 28 à 29 pour la fixation de fractures du tissu cardiaque, comme ancre pour le matériau de suture, comme implant de colonne vertébrale, pour la fermeture et l'assemblage de vaisseaux sanguins et autres, comme endoprothèse, pour le remplissage de cavités ou de trous en cas de défauts tissulaires.

31. Implant selon l'une quelconque des revendications 28 à 30 sous forme d'endoprothèse, **caractérisé en ce qu'**il contient un matériau choisi dans le groupe des cytostatiques.

32. Implant selon l'une quelconque des revendications 28 à 30 sous forme d'une vis, d'une cheville, d'une plaque, d'un écrou, d'une ancre, d'un non-tissé, d'une feuille, d'une membrane ou d'un filet.

33. Procédé pour la fabrication d'un implant selon l'une quelconque des revendications 28 à 32, comprenant les étapes :
(a) séchage d'un copolymère à blocs de formule I ou de formule II selon l'une quelconque des revendications 1 à 17,
(b) transformation du copolymère à blocs à l'état de masse fondue dans une machine de moulage par injection,
(c) injection de la masse fondue de polymère dans un moule présentant les dimensions souhaitées,
(d) refroidissement de la masse fondue de polymère dans le moule de moulage par injection et
(e) isolement du corps moulé du moule de moulage par injection.
